(19) Europäisches Patentamt

European Patent Office

Office européen des brevets

(11) **EP 0 648 499 B1**

(12) **EUROPEAN PATENT SPECIFICATION**

(45) Date of publication and mention
of the grant of the patent:
**31.01.2001 Bulletin 2001/05**

(51) Int Cl.[7]: **A61K 38/02**

(21) Application number: **94116396.6**

(22) Date of filing: **18.10.1994**

(54) **Composition for inhibiting IgE production**

Zusammensetzung zur Hemmung der IgE Produktion

Composition pour l'inhibition de la production d'IgE

(84) Designated Contracting States:
**BE CH DE DK ES FR GB IT LI NL SE**

(30) Priority: **19.10.1993 JP 26135593**

(43) Date of publication of application:
**19.04.1995 Bulletin 1995/16**

(73) Proprietors:
- **Ra, Chisei**
  **Chiba-shi, Chiba 262 (JP)**
- **WelFide Corporation**
  **Osaka (JP)**

(72) Inventors:
- **Yanagihara, Yukiyoshi**
  **Machida-shi, Tokyo (JP)**
- **Ra, Chisei**
  **Chiba-shi, Chiba (JP)**
- **Hirama, Minoru, c/o The Green Cross Corp.**
  **Hirakata-shi, Osaka (JP)**
- **Okumura, Ko**
  **Chiba-shi, Chiba (JP)**

(74) Representative:
**Hansen, Bernd, Dr. Dipl.-Chem. et al
Hoffmann Eitle,
Patent- und Rechtsanwälte,
Arabellastrasse 4
81925 München (DE)**

(56) References cited:
**EP-A- 0 246 967        EP-A- 0 336 554**

- **CHEMICAL ABSTRACTS, vol. 112, no. 23, June 4, 1990, Columbus, Ohio, USA OKAZI T., GOTO J., IWAHASHI K., YAMADA K., SATO M. "Preparation of immuno- globulin E peptide fragments as allergy inhibitors" page 708, column 1, no. 217 552x; & Jpn. Kokai Tokkyo Koho JP,A,01 299 298 (89 299 298)**
- **CHEMICAL ABSTRACTS, vol. 121, no. 13, September 26, 1994, Columbus, Ohio, USA TANIHARA, M., FUJIWARA, C. "Preparation of peptides with binding activity to human immunoglobulin E" page 1086, column 1, no. 158 211w; & Jpn. Kokai Tokkyo Koho JP,A,06 107 685 (94 107 685)**

**Description**

FIELD OF THE INVENTION

The present invention relates to novel use of peptides which are capable of binding to human immunoglobulin E (IgE). More particularly, the invention relates to a use of a soluble fragment of a high affinity immunoglobulin E receptor α-chain (sFcεRIα) which is capable of binding to human IgE for inhibiting production of IgE.

BACKGROUND OF THE INVENTION

B-cells existing in the peripheral lymphoid tissue (spleen and lymph node) carry immunoglobulin on its surface which functions as an antigen-specific receptor. B-cells develop into antibody-producing cells by antigenic stimulation. In this instance, there are two developing systems: one requires help of helper T-cells (T-dependent antibody production); and the other requires no help of them (T-independent antibody production), depending on the types of antigen to mount immune response. It is considered that B-cells involved in these immune reactions can also be divided into subset. When B-cells develop into antibody-producing cells, IgE and the like are produced.

IgE is immunoglobulin bearing allergic reaction to cause hyper-IgE syndrome including bronchial asthma, pollen allergy, atopic dermatitis, non-allergic Kimura's disease (angiolymphoid hyperplasia with eosinophilia), other pulmonary diseases and autoimmune diseases, to which much attention has been paid from the clinical viewpoint.

Immunoglobulin is composed of Fab fragment which forms an antigen binding site and Fc fragment which binds to the corresponding receptor. IgE binds to mast cells and basophils at the Fc fragment and therefore is called "cytotropic antibody". A high affinity immunoglobulin E receptor (FcεRI) is a glycoprotein molecule manifesting on the surface of mast cells and basophils and specifically binds to the Fc fragment of IgE with high affinity (Ka = $10^9$ to $10^{10}$ $M^{-1}$). When a corresponding polyvalent antigen binds to FcεRI-bound IgE, the cells are activated and causes degranulation to release physiologically active substances such as histamine, serotonin, SRS-A (slow reacting substance of anaphylaxis) and the like. These substances function to enhance vascular permeability, contract smooth muscle and promote secretion of mucus to thereby cause type-I allergic symptoms.

Urea-denatured antigen is known to inhibit production of IgE. The urea-denatured antigen inhibits the IgE production reaction by increasing suppressor T cells which suppress helper T cell activity to thereby prevent differentiation of B cells to antibody-producing cells. Suplatast tosilate is also known as a substance capable of inhibiting the IgE production. However, these substances have not been put to practical use due to side effects and other problems.

EP 336 554 relates to peptides having the primary structure Asp-Ser-Asp-Gly-Lys and their use in medicaments for prevention or therapy of type-I allergies.

EP 246 967 relates to plague-forming suppressive lymphokines and medicaments containing said lymphokines.

JP 89 294 298 relates to the preparation of immunoglobulin E peptide fragments as allergy inhibitors.

J. Biol. Chem. 266, 4 (1991), 2639-2646, relates to the characterization of truncated α-chain products of high affinity receptor for IgE.

The present inventors have developed, as a prophylactic and therapeutic composition for allergic diseases, a peptide capable of specifically binding to the Fc fragment of IgE with high affinity on the cell membrane of mast cells and basophils, from which the type I allergic reaction mechanism originates, to inhibit the binding of IgE to FcεRI. This peptide is capable of binding to IgE, but has not been known to directly act on B cells and inhibit production of IgE in B cells.

SUMMARY OF THE INVENTION

An object of the present invention is to provide a clinically useful composition for inhibiting production of IgE which arrests type I allergic reactions at the rudiment.

The inventors of the present invention have found after much research directed to the above object that the prophylactic administration of a peptide which is capable of binding to human IgE inhibits the IgE production to control the onset of type I allergic diseases. Even when the peptide is administered after type I allergic symptoms are shown, the symptoms can be ameliorated due to the inhibition of the IgE production.

Having been developed on the basis of the above findings, the present invention relates to a use of a peptide which is capable of binding to human IgE for inhibiting production of IgE.

BRIEF DESCRIPTION OF THE DRAWINGS

Fig. 1 illustrates the structure of a plasmid designated as FcεRI/pMT1.

Fig. 2 illustrates the scheme for constructing the plasmid designated as pTT06.

Fig. 3 is a graph showing inhibitory effect of purified sFcεRIα on the IgE production in human peripheral blood mononuclear cells (PBMC) stimulated with IL-4 (A), in B cells stimulated with IL-4 and anti-CD40 monoclonal antibody (B) or IL-4 and hydrocortisone (HC). In Fig. 3, ∗ means that the difference is statistically significant ($p < 0.05$, paired t-test).

Fig. 4 is an electrophoretic pattern showing the effect of purified sFcεRIα on expression of CεmRNA in cultured B cells. In Fig. 4, lanes 1 and 4 stand for control, lanes 2 and 5 for 10 ng/ml of sFcεRIα and lanes 3 and 6 for 100 ng/ml of sFcεRIα.

Fig. 5 is a graph showing the effect of purified sFcεRIα on production of IgG (A), IgM (B) and IgA (C) in peripheral blood mononuclear cells (PBMC) stimulated with pokeweed mitogen (PWM).

## DETAILED DESCRIPTION OF THE INVENTION

The term "peptide which is capable of binding to human IgE" as used herein means a peptide which specifically binds to human IgE to inhibit its function. Any peptide is usable in the present invention as long as it shows about 80% IgE inhibition in the IgE inhibition assay as described below and is a peptide containing a soluble fragment of such a high affinity immunoglobulin E receptor α-chain (sFcεRIα) which are capable of binding to human IgE.

A high affinity immunoglobulin E receptor (FcεRI) is a glycoprotein having a tetrameric structure consisting of an α-chain, a β-chain and two disulfidized γ-chains. It has been reported that only the extracellular region of the α-chain is involved in the high-affinity binding to IgE [Blank, U. et al., J. Biol. Chem., 266, 2639 (1991)].

FcεRIα stands for the α-chain of said FcεRI, and sFcεRIα stands for the extracellular region of the α-chain.

A peptide which is capable of binding to human IgE for use in the present invention, need only contain at least the extracellular region of the α-chain, viz. sFcεRIα, and both FcεRIα and FcεRI may likewise be employed with success. Mutants artificially derived by a genetic engineering technique and a derivative for drug delivery system, for example, the conjugate with albumin, can also be used if the mutant is capable of binding to human IgE, but sFcεRIα is particularly desirable. Preferably, the peptides are obtained and derived from humans.

sFcεRIα can be produced by tissue culture or by genetic engineering. Blank U. et al. (*vide supra*) reported that sFcεRIα is produced by gene engineering techniques using a dihydrofolate reductase (DHFR)-deficient CHO cells.

The present inventors have succeeded in production of sFcεRIα in large quantity by transfecting an animal cell with a plasmid (1) carrying a DNA coding for sFcεRIα with a promoter capable of controlling the expression thereof in animal cells and a DHFR gene with a urokinase (UK) promoter or transfecting an animal cell with both a plasmid (2) carrying a DNA coding for sFcεRIα with a promoter capable of controlling the expression thereof in animal cells and a plasmid (3) carrying a DHFR gene with a UK promoter, and culturing the animal cells (cf. Reference Example below).

The production of sFcεRIα by genetic engineering may be carried out by using *Escherichia coli* or yeasts instead of the animal cells.

The composition for inhibiting production of IgE comprising a peptide capable of binding to human IgE according to the present invention can be given to patients suffering from hyper-IgE syndrome such as non-allergic Kimura's disease, other pulmonary diseases or autoimmune diseases as either systemic therapy or topical therapy.

For systemic treatment, the peptide is dissolved in physiological saline for injection or distilled water for injection and administered parenterally, preferably intravenously. The dosage form may be a liquid or a lyophilizate and can be manufactured by the methods well known in the art.

For topical administration, various dosage forms for external application to the skin or mucosa such as ocular mucosa, nasal mucosa, bronchial mucosa, etc. can be utilized. Examples of the dosage form include spray, ophthalmic solutions, nasal solutions and ointments.

Any of such pharmaceutical preparations can be manufactured by the known manufacturing method.

For example, a spray can be manufactured by dissolving the peptide capable of binding to human IgE in an appropriate solvent in the conventional manner and putting it in a sprayer to serve as aerosol for commonly employed inhalation therapy.

An ophthalmic or nasal solution can be manufactured by dissolving the active ingredient peptide which is capable of binding to human IgE in distilled water for injection, adding any auxiliary agent required, such as a buffer, isotonizing agent, thickener, preservative, stabilizer, surfactant, antiseptic, etc., and adjusting the mixture to pH 4 to 9. A nasal spray can be manufactured for example by the method described in JP-A-63-101318.

The ointment which may be used in the present invention is preferably a gel ointment. Such an ointment can be manufactured, for example, by mixing the active ingredient peptide which is capable of binding to human IgE with a gel base prepared by using a carboxyvinyl polymer and, as a basic thickener, sodium hydroxide or the like and, after addition of auxiliary agents as necessary, adjusting the mixture to pH 4 to 9.

The composition can be used for both prophylaxis and symptomatic control. The dosage is dependent on the type of disease and the patient's age, sex and condition. In normal human serum, IgE circulates at a concentration of about 3 ng/ml, which is based on a molecular weight of 185,000, corresponding to about $10^{10}$ IgE molecules per ml of serum. In serum of patients suffered from allergic diseases, the IgE concentration raises to 100 to 10,000 times as high as that in normal human serum. For example, the IgE concentration in serum of patients suffered from hay fever, allergic rhinitis or atopic asthma is about 300 ng/ml ($10^{12}$ molecules/ml), and that in atopic dermatitis patient is about 30 μg/ml ($10^{14}$ molecules/ml). For prophylactic treatment, it is preferable to administer at least twice the amount of IgE corresponding to the exogenous factor. For symptomatic control, it is preferable to use a massive dose soon after onset, preferably at least 10-fold as great a dose relative to the amount of IgE corresponding to the exogenous factor. The composition may be administered in an amount of not less than 100 μg/kg for prophylactic treatment and not less than 500 μg/kg for nosotropic therapy, in terms of the active ingredient. The composition of the present invention can be used in combination with other compositions.

The peptide capable of binding to human IgE according to the present invention binds to membrane-bound IgE on both activated T-dependent or T-independent B cells and is taken into the cell to specifically inhibit the IgE production, thereby arresting type I allergic reactions at the rudiment. Thus, the present invention provides a clinically useful composition for inhibiting production of IgE. Since the composition of the present invention specifically inhibits the production of IgE at the rudiment without inhibiting the production of other immunoglobulins, the sufficient prophylactic and/or therapeutic effect can be achieved with reduced dosage times and with relieved side effects, as compared with the conventionally used IgE production inhibitors.

The following reference, test, and working examples illustrate the invention in further detail. However, they are not to be construed as limiting the scope of the invention.

REFERENCE EXAMPLE 1

Production of sFcεRIα

[I] Construction of FcεRI/pMT1

[Construction of a plasmid using a DNA coding for the human sFcεRIα chain with a promoter (SV40) capable of controlling the expression of said chain in an animal cell]

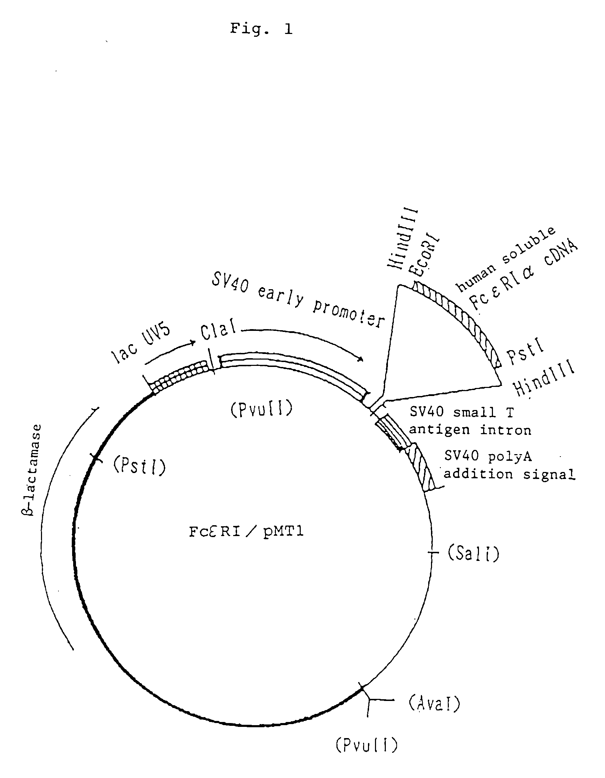

An expression vector FcεRI/pMT1 (Fig. 1) was constructed by inserting human FcεRIα leader sequence-human sFcεRIα gene into plasmid pko [K.V. Doren et al., J. Virol., 50, 606 (1984)] in accordance with the method of Blank et al. [Blank, U. et al., J. Biol. Chem., 266, 2639 (1991)]. A DNA sequence coding for said human sFcεRIα and an amino acid sequence deduced therefrom are disclosed in Nucleic Acids Research, 16(8), 3584 (1988).

[II] Construction of pTT06

[Construction of a plasmid using a DNA coding for dihydrofolate reductase (DHFR) with the urokinase (UK) promoter]

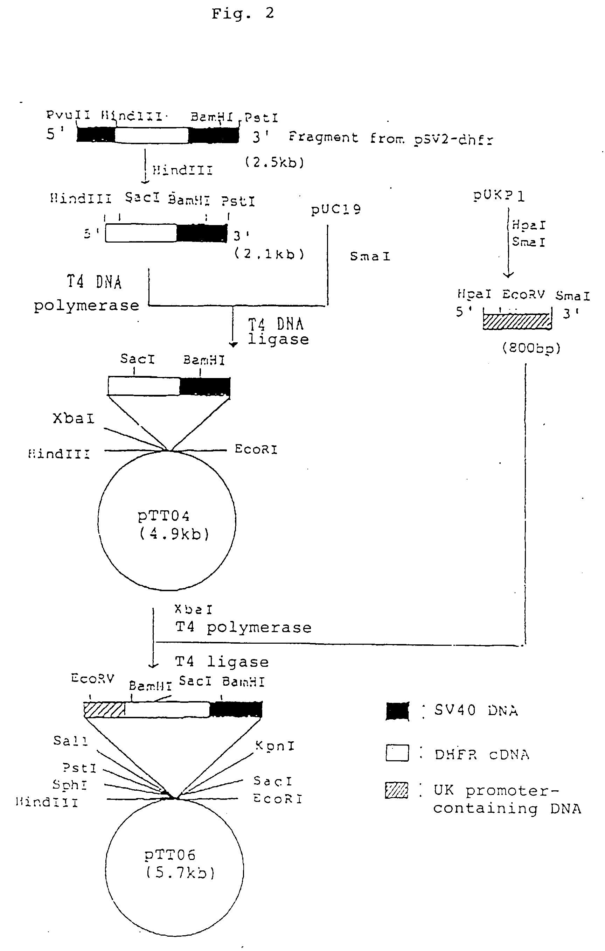

A plasmid, pTT06, containing the UK promoter, DHFR cDNA and SV40 polyA was constructed in accordance with the method described in Biosci. Biotech. Biochem., 56(4), 600-604 (1992) (cf. Fig. 2).

[III] Introduction into animal cells and establishment of a high-productivity cell strain

(i) Materials:

Plasmid DNA

FcεRI/pMT1: The plasmid produced by incorporating the human FcεRIα chain leader sequence and the sFcε–RIα chain gene into plasmid pko.

pTT06: The plasmid containing a DHFR gene expression unit placed under the control of the UK promoter (5' upstream regulatory region comprising about 800 bp and containing the urokinase gene transcription initiation site).

Cells: The cell line CHO DXB-11 (DHFR-deficient strain).

Prepared and multiplied by the method described by G. Urlaub et al. in Proc. Natl. Acad. Sci. USA, 77, 4216-4220 (1980).

Methotrexate (MTX): A 2 mM stock solution was prepared by dissolving (+)Amethopterin (Sigma) in 0.14 M NaCl + 0.02 M HEPES (Nacalai Tesque). This solution was added to the medium to a predetermined concentration.

(ii) Procedure

(1) DNA introduction and transfectant supernatant assaying for expression

DHFR-deficient CHO DXB-11 cells subcultured using Eagle's MEM-$\alpha$ supplemented with 10% FCS were detached from the dish by treatment with trypsin (0.25%) and suspended in Hanks' solution to a density of $10^7$ cells/ml. The plasmid DNAs (1 $\mu$g of pTT06 and 40 $\mu$g of Fc$\epsilon$RI/pMT1) were simultaneously introduced into 5 x $10^6$ cells (0.5 ml of the suspension) by electropolation. After cultivation in Eagle's MEM-$\alpha$ (ribonucleic acid- and deoxyribonucleic acid-free) supplemented with 10% FCS, the resultant colonies were picked and cultured. The spent culture medium of the transfectants was assayed for human sFc$\epsilon$RI$\alpha$ chain activity by the method described below under (2) and strains showing high activity were used for DNA amplification using MTX.

(2) Assaying of the human sFc$\epsilon$RI$\alpha$ chain

The human sFc$\epsilon$RI$\alpha$ chain concentration was determined in terms of the degree of inhibition of binding of $^{125}$I-labeled mouse IgE to Fc$\epsilon$RI occurring on a rat basophilic cell line. The materials and procedure used were as follows.
(Materials)
Cells: The rat basophilic cell line RBL-2H3 [Barsumian, B.L. et at., Eur. J. Immun., 11, 317-323 (1981)] was cultivated in Eagle's MEM containing 10% FCS, 100 U/ml penicillin and 100 $\mu$g/ml streptomycin.
IgE: Anti-dinitrophenyl mouse monoclonal IgE [Hi-DNP-E-26-82; Liu, F.T. et al., J. Immunol., 124, 2728-2737 (1980)] or anti-trinitrophenyl (TNP) mouse monoclonal IgE was used. The anti-TNP mouse monoclonal IgE was purified from the culture supernatant of the hybridoma IGELb4 (ATCC No. TIB141) or mouse ascitic fluid.
Iodine-labeling of IgE: The Bio-Rad Enzymobeads method was used. A mixture was prepared from 50 $\mu$l of 0.2 M phosphate buffer (pH 7.2), 10 $\mu$l of mouse monoclonal IgE (2.14 mg/ml), 50 $\mu$l of Enzymobeads reagent, 25 $\mu$l of Na$^{125}$I (95 MBq) and 25 $\mu$l of a 1% aqueous solution of $\beta$-D-glucose. The reaction was carried out at room temperature for 15-20 minutes. After admixing with 150 $\mu$l of phosphate-buffered physiological saline containing 10 mg/ml tyrosine, 10% glycerol and 0.1% xylene cyanole, protein recovery was effected by gel filtration using a PD-10 column (Pharmacia).

(Procedure)

IgE binding assay:

1) Since CHO cells are maintained using the 10% FCS-supplemented Eagle's MEM-$\alpha$ (ribonucleic acid- and deoxyribonucleic acid-free), samples were prepared by diluting with this culture medium (125-250 $\mu$l).
2) $^{125}$I-Labeled IgE diluted with Dulbecco's phosphate buffered physiological saline supplemented with 3% bovine serum albumin and 0.1% NaN$_3$ (PBS/3% BSA) was added to each sample prepared in 1). The whole volume was 500 $\mu$l and the concentration of $^{125}$I-labeled IgE was 100 ng/ml. This mixture was incubated at room temperature for 3-6 hours.
3) To said mixture was added 50 $\mu$l of a suspension of RBL-2H3 cells (3-8 x $10^7$ cells/ml) in PBS/3% BSA, followed by 1-2 hours of incubation on ice. In an unlabeled IgE group used for assessing nonspecific adsorption of labeled IgE on cells, a mixture of 150 $\mu$l of the RBL-2H3 cell suspension and 15 $\mu$l of 2.1 mg/ml unlabeled IgE was prepared in advance. This mixture (55 $\mu$l) was added to the mixture prepared in step 2).
4) Cells were caused to settle as a sediment by centrifugation (1,000 rpm, 5 minutes) and the supernatant was discarded.
5) The cells were rinsed once with PBS/3% BSA and the bound radio-activity was measured using a gamma counter.
6) The cpm value obtained by adding Eagle's MEM-$\alpha$ (ribonucleic acid- and deoxyribonucleic acid-free) supplemented with 10% FCS was used as a control (the additive-free case) and the percent binding inhibition was calculated as follows:

$$\text{Binding inhibition (\%)} = \frac{\text{Radioactivity in additive-free case - Radioactivity in test group}}{\text{Radioactivity in additive-free case - Radioactivity in IgE-added group}} \times 100$$

(3) Amplification of the inserted gene using MTX

Dishes (10 cm) containing 8-10 ml of a selective medium containing 10 nM MTX were inoculated with a human sFc$\epsilon$RI$\alpha$ chain-producing strain obtained as described in (1) to a density of 5-10 x $10^5$ cells/dish. Cultivation was continued for 2-4 weeks while performing medium exchange at intervals of about 3 days. Thereby, a sufficient number of 10 nM MTX-resistant cells were obtained. The cells were transferred to a medium at the next MTX concentration level. In this way, the MTX concentration was increased stepwise for gene amplification, starting from 10 nM MTX, to 50 nM, 100 nM, 200 nM, 500 nM, 1 μM, 2 μM, 4 μM and 10 μM.

(4) Cloning of human sFcεRIα chain producers

Among the human sFcεRIα chain producers obtained after gene amplification using MTX as described in (3), several strains were subjected to cloning by the limiting dilution method. Thus, cells resistant to each concentration of MTX were cultured in Eagle's MEM-α (ribonucleic acid- and deoxyribonucleic acid-free) supplemented with 10% FCS and 2-10 μM MTX and distributed into the wells of a 96-well plate. Cells were recovered from wells showing cell proliferation and transferred to a 24-well plate and then, further, to a 10 cm dish. At the stage of confluence, the production of the human sFcεRIα chain in each supernatant was determined.

[IV] Purification of the human sFcεRIα chain

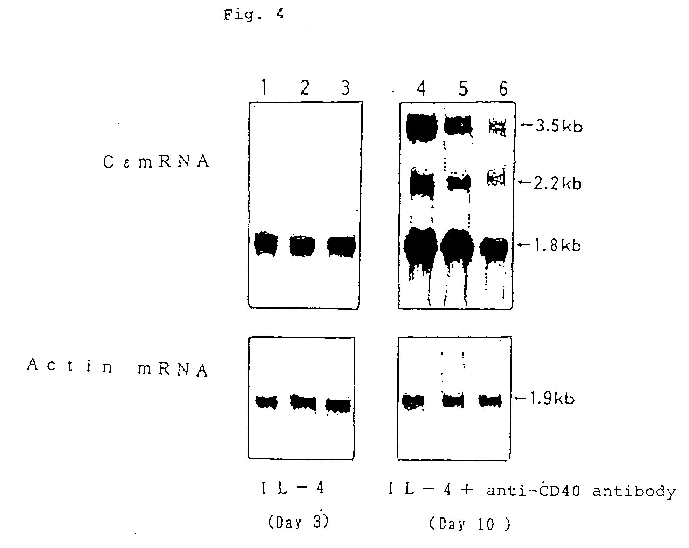

To 983 ml of the culture supernatant derived from a cloned cell strain showing a relatively high production of the human sFcεRIα chain was added a 1/20 volume of 1 mM Tris, pH 8.0, together with 10 mM banzamidine, 10 mM ethylenediaminetetraacetic acid and 0.02% sodium azide. The mixture was filtered (0.22-μm filter) and the filtrate was purified using an IgE-Sepharose 4B column [prepared by binding 10 mg of anti-trinitrophenyl IgE purified from a mouse hybridoma IGELb4 (ATCC No. TIB141) culture supernatant to 1 g of activated CH Sepharose (Pharmacia) in 0.1 M sodium hydrogen carbonate]. The column was washed with PBS containing 0.02% sodium azide. The protein bound to the column was eluted with an eluant comprising 0.2 M acetic acid, 0.2 M sodium chloride and 0.02% sodium azide, pH 2.8. The thus purified human sFcεRIα chain was dialyzed against 10 mM ammonium bicarbonate, and 200 μl of the dialyzate was dried in SpeedVac and checked by SDS electrophoresis. Upon staining with Coomassie Brilliant Blue, a distinct band was detected at a position corresponding to a molecular weight of about 50 kDa, without any other protein band, under reducing conditions as well as under nonreducing conditions (Fig. 4). This protein had an activity of inhibiting the binding of radio-labeled IgE to basophilic cells (RBL-2H3 cells; vide supra). The concentration of such purified human FcεRIα chain was determined by the method of Bradford [Bradford, M. M., Anal. Biochem., 72, 248 (1976)].

EXAMPLE 1

Composition for administration by injection or spraying

In distilled water for injection were dissolved 100 mg of the purified human sFcεRIα and 100 mg of glucose to give a purified human sFcεRIα concentration of 2 mg/ml. The solution was filtered using a 0.45-μm membrane filter and the filtrate was aseptically distributed into 5-ml vials, followed by nitrogen gas charging and tight closure to give a composition for intravenous injection or for spraying.

EXAMPLE 2

Ophthalmic solution

In distilled water for injection were dissolved 500 mg of the purified human sFcεRIα, 50 g of sorbitol and 20 mg of methyl parahydroxybenzoate. The pH was adjusted to 6.5 with phosphate buffer and the whole volume was made to 1,000 ml. The solution was filtered using a 0.45 μm membrane filter and the filtrate was aseptically distributed into eye drop bottles. Thus was prepared an ophthalmic solution.

EXAMPLE 3

Composition for nasal administration

A composition for nasal administration was prepared in the same manner as in Example 2.

EXAMPLE 4

Ointment

To 25.0 parts by weight of a 2% aqueous solution of a carboxyvinyl polymer (Carbopol 940, product of Goodrich) was added gradually 25 parts by weight of a 2% aqueous solution of sodium hydroxide with stirring. Further stirring gave a gel-like composition.
A solution of 2 parts by weight of the purified human sFcεRIα in an appropriate amount of purified water was added to said gel-like composition, followed by further addition of purified water to make the whole amount 100 parts by weight. The subsequent uniform stirring gave an ointment.

TEST EXAMPLE 1

Inhibitory effect of purified human sFcεRIα on IgE production

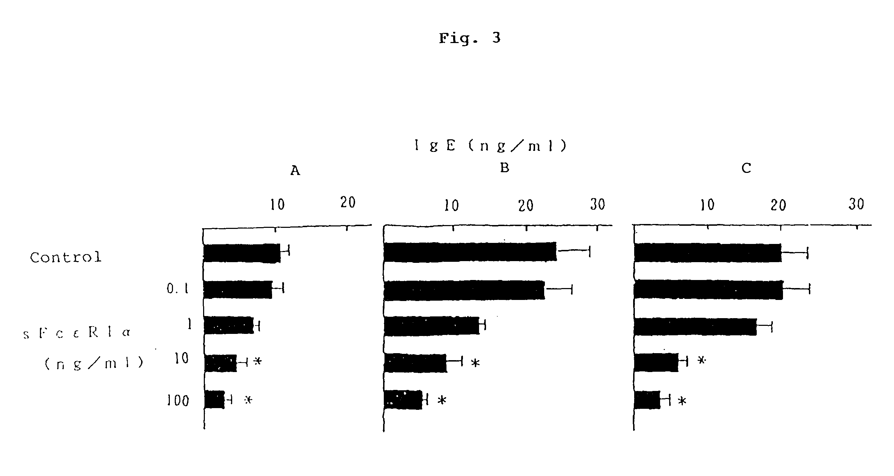

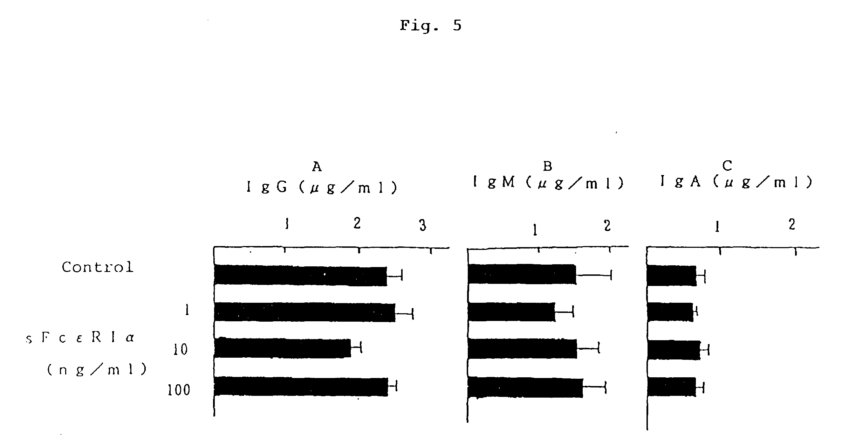

Peripheral blood mononuclear cells (PBMC) derived from a healthy adult were treated with acidic buffer (pH 4.0) and adhesive monocytes were removed using a Petri dish. Then, B cells were isolated and purified by the rosette method using sheep erythrocytes and the panning method using anti-CD3 monoclonal antibody. PBMC was stimulated with interleukin-4 (IL-4, 100 U/ml) or pokeweed mitogen (PWM, 0.1 μg/ml) and B cells were stimulated with a combination of IL-4 and anti-CD40 monoclonal antibody (1 μg/ml) or a combination of IL-4 and hydrocortisone (HC, 1 μM), for 14 days. Each class of immunoglobulin produced in each culture supernatant was assayed by isotype-specific solid phase sandwich RIA. IgE assay was carried out using two anti-IgE monoclonal antibodies whose binding activity to IgE was not inhibited concentration-dependently by purified human sFcεRIα.
After stimulation of B cells with IL-4 for 3 days or with a combination of IL-4 and anti-CD40 monoclonal antibody for 10 days, total RNA was extracted from each group of cells and subjected to Northern blotting using cDNA probe specific for Cε2-Cε4 to examine as to how CεmRNA was expressed. Markers on the B cell surface were examined using a fluorescence activated cell sorter (PACS). The binding test of purified human sFcεRIα to IgE was carried out using anti-sFcεRIα monoclonal antibody which did not bind to IgE.
As a result, the IgE production in PBMC stimulated with IL-4 and B cells stimulated with a combination of IL-4 and anti-CD40 monoclonal antibody or a combination of IL-4 and HC was inhibited concentration-dependently by 1 to 100 ng/ml of purified sFcεRIα. The IgE production was statistically significantly inhibited by purified sFcεRIα at a concentration ranging from 10 to 100 ng/ml ($p < 0.05$, paired t-test) (Fig. 3). Thus, from the results that purified sFcεRIα inhibited the IgE production in both of T cell-dependent system and T cell-independent system, it was confirmed that a target cell of sFcεRIα was B cell. In the case that B cells were stimulated with a combination of IL-4 and anti-CD40 monoclonal antibody, purified human sFcεRIα inhibited expression of secretory CεmRNA (2.2 kb) at a concentration of from 10 to 100 ng/ml. On the other hand, in the case that B cells were stimulated with IL-4 alone, sFcεRIα showed no effect on the expression of germline CεmRNA (1.8 kb) (Fig. 4).
It was also found that purified human sFcεRIα did not affect the expression of various markers on the surface of the B cell stimulated with IL-4, including a low affinity IgE receptor FcεRII (CD23), CD40, HLA-DR, IgM and IL-4 receptor and it did not inhibit liberation of soluble CD23 (sCD23). Further, the inhibitory effect of anti-CD23 monoclonal antibody against the IgE production in B cells was not affected by purified human sFcεRIα. However, purified human sFcεRIα reduced the IgE production of B cells enhanced by 29 kd sCD23 to the normal level or less.
Furthermore, purified human sFcεRIα did not bind to T cells, B cells, NK cells and monocytes derived from a healthy adult as well as IgE non-producing B cell lines (e.g., RPMI1788, RPMI8866, Daudi), while it bound to IgE-producing human myeloma cell (U266). The excess amount of IgE dissociated the bonding between purified human sFcεRIα and the myeloma cell.
On the other hand, purified human sFcεRIα did not prevent the production of IgG, IgM and IgA in PBMC stimulated with PWM (Fig. 5).
From these results, it was found that purified human sFcεRIα was a novel molecule capable of specifically inhibiting production of IgE class antibodies.

**Claims**

1. The use of a peptide containing a soluble fragment of the high-affinity immunoglobulin E receptor α-chain for the preparation of a composition for the prevention or treatment of hyper-IgE syndrome other than anaphylactic shock, pollen allergy, atopic dermatitis and bronchial asthma.

**2.** The use according to claim 1, wherein said hyper-IgE syndrome is non-allergic Kimura's disease.

**3.** The use according to claim 1, wherein said hyper-IgE syndrome is pulmonary disease.

**4.** The use according to claim 1, wherein said hyper-IgE syndrome is an autoimmune disease.

**5.** The use according to any one of claims 1 to 4, wherein said soluble fragments of the high-affinity immunoglobulin E receptor α-chain is of human origin.

**Patentansprüche**

**1.** Verwendung eines Peptids, das ein lösliches Fragment der α-Kette des hochaffinen Immunoglobulin E-Rezeptors enthält, zur Herstellung einer Zusammensetzung zur Vorbeugung oder Behandlung von einem anderen Hyper-IgE-Syndrom als anaphylaktischem Schock, Pollenallergie, atopischer Dermatitis und Bronchialasthma.

**2.** Verwendung gemäss Anspruch 1, wobei das Hyper-IgE-Syndrom die nicht-allergische Kimura-Krankheit ist.

**3.** Verwendung gemäss Anspruch 1, wobei das Hyper-IgE-Syndrom eine Lungenkrankheit ist.

**4.** Verwendung gemäss Anspruch 1, wobei das Hyper-IgE-Syndrom eine Autoimmunkrankheit ist.

**5.** Verwendung gemäss einem der Ansprüche 1 bis 4, wobei die löslichen Fragmente der α-Kette des hochaffinen Immunoglobulin E-Rezeptors humanen Ursprungs sind.

**Revendications**

**1.** Utilisation d'un peptide contenant un fragment soluble de la chaîne α d'un récepteur d'immunoglobulines E de grande affinité pour la préparation d'une composition destinée à la prévention ou au traitement d'un syndrome d'hyper-IgE autre que le choc anaphylactique, l'allergie au pollen, l'eczéma atopique et l'asthme bronchique.

**2.** Utilisation selon la revendication 1, dans lequel ledit syndrome d'hyper-IgE est la maladie de Kimura non allergique.

**3.** Utilisation selon la revendication 1, dans lequel ledit syndrome d'hyper-IgE est une maladie pulmonaire.

**4.** Utilisation selon la revendication 1, dans lequel ledit syndrome d'hyper-IgE est une maladie auto-immune.

**5.** Utilisation selon l'une quelconque des revendications 1 à 4, dans laquelle ledit fragment soluble de la chaîne α du récepteur d'immunoglobulines E de grande affinité est d'origine humaine.

Fig. 1

Fig. 2

Fig. 3

Fig. 4

C ε m R N A

←3.5kb

←2.2kb

←1.8kb

Actin mRNA

←1.9kb

I L − 4

(Day 3)

I L − 4 + anti-CD40 antibody

(Day 10 )

Fig. 5